# EUROPÄISCHE PATENTANMELDUNG

(11) **EP 0 734 727 A2**
(43) Veröffentlichungstag der Anmeldung: **02.10.1996**
(21) Anmeldenummer: 96105121.6
(22) Anmeldetag: 29.03.1996
(51) Int. Cl.: A61K 35/78

(54) **Verwendung von Teebaumöl zur Herstellung von Arznei- bzw. Pflegemitteln, sowie Arznei- bzw. Pflegemittel mit Teebaumöl**

(30) Priorität: 29.03.1995 DE 19511429
(71) Anmelder: Linnhoff, Gerhard, 25557 Hademarschen (DE)
(72) Erfinder: Linnhoff, Gerhard, 25557 Hademarschen (DE)
(74) Vertreter: Heun, Thomas

(57) **Zusammenfassung**

Es werden eine Verwendung von Teebaumöl zur Herstellung eines Arznei- bzw. Pflegemittels zur Vorbeugung gegen und zur Behandlung bei einen/m Befall mit Flöhen, Zecken, Milben, Haarlingen, Läusen und anderen blutsaugenden Parasiten bei Tieren, insbesondere Haustieren wie Hunden und Katzen, sowie saugenden Parasiten, insbesondere Blattläusen bei Pflanzen, sowie verschiedene Arznei- bzw. Pflegemittel mit Teebaumöl beschrieben.

## Beschreibung

Die Erfindung betrifft die Verwendung von Teebaumöl (Tea Tree) zur Herstellung von Arznei- bzw. Pflegemitteln auf biologischer Basis, sowie Arznei- bzw. Pflegemittel mit Teebaumöl, insbesondere für Tiere.

Ausgangsbasis für die Gewinnung von Teebaumöl ist der Teebaum (wissenschaftlicher Name: melaleuca alternifolia), der zur Gattung der Myrtengewächse gehört und mit über 100 Arten in Australien und Tasmanien weit verbreitet ist. Er wird bis zu 7 Meter hoch, hat eine viellagige, leicht ablösbare Rinde und weiche, nadelartige Blätter, die dicht zusammenstehen und einen sehr aromatischen Duft freisetzen.

Aus seinen Blättern wird das Teebaumöl (Tea Tree) gewonnen, bei dem es sich um eine farblose bis leicht gelbliche Flüssigkeit handelt, die einen charakteristischen Geschmack und Geruch aufweist. Das entsprechende ätherische Öl entsteht durch Dampfdestillation, wobei eine Tonne Blätter etwa 10 Liter Öl ergeben können. Die Blätter können alle 18 Monate abgeerntet werden und wachsen dann wieder neu nach. Aus den Blättern kann auch ein würziger Tee gekocht werden. Teebaumöl ist gewebeschonend und vollkommen ungiftig für Menschen und Haustiere.

Die Hauptbestandteile des Teebaumöls sind: Cineol 1,8 mit etwa 9,1 Vol%, P Cymene mit etwa 16,1 Vol%, Terpinen-4-ol mit etwa 31,2 Vol% und Alpha Terpineol mit etwa 3,5 Vol%.

Arznei- bzw. Pflegemittel, die Teebaumöl enthalten, sind für verschiedene Anwendung bei Menschen und Tieren bereits bekannt geworden.

Die DE-OS 29 20 036 offenbart z.B. zur Behandlung von Hämorrhoiden und Verletzungen bei Tieren eine Creme, die neben Goldsiegel- und Collinsonia-Wurz auch Teebaumöl enthält.

Aus der EP 0 594 926 ist ein Mittel zur Härtung von hornartigen Körperteilen von Säugetieren bekannt, in dem in einem Trägeröl auf pflanzlicher Basis neben Zitronenöl u.a. auch Teebaumöl gelöst sein kann.

Die WO 91/10364 betrifft ein Mittel zur Vorbeugung und Behandlung bei Brustdrüsenentzündung und Entzündungen des Euters von Milchkühen. Das Mittel kann eine Salbe, ein Spray oder eine Tinktur sein und enthält als Wirkstoff auch Teebaumöl.

Ferner wird dort beschrieben, daß dieses Mittel auch eine Insekten vertreibende Wirkung hat, wenn Teebaumöl in einer Zusammensetzung mit einem Anteil von 1 bis 6% vorhanden ist, und daß es konservierend, fungizid und bakterizid ist.

Die US-PS 5.215.748 betrifft ein Mittel und ein Verfahren zur Vorbeugung und Behandlung bei Bläschen durch Herpes Simplex Viren beim Menschen, welches auch Teebaumöl aufweist.

Aus der US-PS 5.096.709 ist ein Mittel zur Entspannung von Muskeln sowie zur Linderung von Schmerzen bekannt, welches neben anderen Bestandteilen auch 5 bis 10% Teebaumöl enthält.

In der US-PS 5.009.890 wird ein Mittel zur Behandlung von Brandwunden beschrieben, dessen aktive Bestandteile Wasser und eine Teebaumölmischung (Terpene und Terpinole), auch in Kombination mit reinem Teebaumöl, sind.

Die US-PS 4.925.655 offenbart schließlich ein in Wasser lösliches Puder mit zahlreichen Inhaltsstoffen, unter anderem Teebaumöl mit einem Anteil von zwischen 0,5 und 10 Gewichts%. Dieses Puder ist zur Herstellung eines Mundwassers vorgesehen, das zur Reinigung der Zähne, insbesondere zur Beseitigung von Zahnbelag dienen soll.

Arznei- und Pflegemittel müssen zahlreiche und zum Teil sehr strenge Anforderungen erfüllen. Im Idealfall sollen sie einerseits z.B. bei unsachgemäßem Gebrauch keine ernsten Gefahren für Mensch oder Tier darstellen, andererseits sollen sie insbesondere bei Tieren als Allheilmittel bei leichten Erkrankungen und Blessuren helfen, die nicht der Behandlung durch einen Tierarzt bedürfen. Die zahlreichen bekannten Mittel dieser Art haben häufig den Nachteil, daß sie jeweils nur für ganz bestimmte Anwendungen vorgesehen sind und/oder sogar chemische Mittel darstellen, die bei Mensch und Tier Allergien hervorrufen und bei Überdosierung oder falscher Anwendung zu ernsten Gesundheitsgefährdungen führen können. Dies betrifft z.B. auch die in chemischen Injektiziden häufig vorkommenden Stoffe Propoxur und Permethrin, die eine Belastungsdauer von 5 Monaten bzw. 1 Jahr und länger aufweisen (vgl. Bundesgesundheitsblatt 12/1992, Seite 608).

Ein schwerwiegendes Problem ist insbesondere die Bekämpfung von blutsaugenden Parasiten wie Flöhen, Zecken, Milben, Haarlingen, Läusen usw., da sie sich an ihrem Wirt festklammern, Krankheiten übertragen und Infektionen hervorrufen können und nicht wie die vergleichsweise relativ harmlosen Insekten im klassischen Sinne mit üblichen Mitteln beseitigt werden können.

Der Erfindung liegt die Aufgabe zugrunde, nach Arznei- bzw. Pflegemitteln auf biologischer Basis zu suchen, mit denen nicht nur eine wirksame Bekämpfung dieser blutsaugenden Parasiten möglich ist und die bei unsachgemäßem Gebrauch keine ernsten Gefahren für Mensch oder Tier darstellen, sondern die insbesondere bei Haustieren auch ein möglichst weites Wirkungsspektrum im Sinne eines Allheilmittels aufweisen, dessen Anwendung durch den Tierhalter selbst erfolgen kann.

Gelöst wird diese Aufgabe gemäß Anspruch 1 durch die Verwendung von Teebaumöl (Tea Tree) zur Herstellung eines Arznei- bzw. Pflegemittels zur Vorbeugung gegen und zur Behandlung bei einen/m Befall mit Flöhen, Zecken, Milben, Haarlingen, Läusen und anderen blutsaugenden Parasiten bei Tieren, insbesondere Haustieren wie Hunden und Katzen, welches sich dadurch auszeichnet, daß es Teebaumöl in reiner Form zur äußeren Anwendung aufweist.

Die Wirkungsweise beruht darauf, daß das Mittel in reiner Form, d.h. im wesentlichen ohne Zusatzstoffe durch leichtes Einreiben in die Haut eines Tieres eindringt und in dessen Blutbahn gelangt und dann von den das Blut saugenden Parasiten aufgenommen wird, was dazu führt, daß diese daran zugrunde gehen. In Kombination damit und als Nebeneffekt des Einreibens (oder durch zusätzliche Gabe einiger Tropfen des Mittels in das Fell des Tieres) verdampft eine gewisse Menge Teebaumöl, was insbesondere zur Folge hat, daß Parasiten davon abgehalten werden, das Tier erneut zu befallen. Wird das Mittel schließlich direkt auf die Parasiten aufgebracht, was i.a. nur bei einer Zecke sinnvoll ist, so wird diese durch Austrocknen, Ersticken sowie Zersetzen des Chitinpanzers unmittelbar vernichtet.

Ein besonderer Vorteil des erfindungsgemäßen Arznei- bzw. Pflegemittels besteht darin, daß es sich tatsächlich bei zahlreichen anderen Anwendungen als wirksam erwiesen hat. Es ist nicht nur zur Bekämpfung der genannten saugenden Parasiten, sondern auch zur Behandlung von Zahnfleischentzündungen, zur Kariesprophylaxe bei Pulpaöffnung, zur Tamponierung nach einer Zahnextraktion, bei Mundgeschwüren, Ausschlägen, Krallenbruch bzw. Kralleninfektion, kleinen Schnitt- und Schürfwunden (Antiseptikum), bei Pyodermien, zur Fistelbehandlung und Scheiden- bzw. Gebärmutterspülung, sowie bei Warzen, Blasen und Insektenstichen bzw. -bissen geeignet, wobei das Auftragen vorzugsweise mit einem Wattestäbchen erfolgt. Ferner können Ektoparasiten bei Pferden, Schweinen, Kühen, Schafen und Ziegen behandelt werden, ebenso wie Huf- und Klauengeschwüre, Ballenfäule, Strahlfäule, Moderhinke. Es kann auch als Mastistherapeutikum dienen und ist wirksam bei einer unspezifischen Reiztherapie bei der Behandlung eines Sommerexzems bei Pferden.

Vorzugsweise wird solches Teebaumöl verwendet, bei dem der Gehalt des Cineol 1,8 unter 4 Vol% und der Gehalt des Terpinen-4-ol über 34 Vol% liegt. In diesem Fall ist die Wirksamkeit besonders gut.

Verschiedene Zusatzstoffe haben zur Folge, daß das erfindungsgemäße Mittel für bestimmte oder zusätzliche Anwendungen und/oder Tierarten besonders geeignet ist:
Es kann z.B. ein als Duftstoff wirkender, geringer Anteil eines anderen ätherischen Öles zugemischt werden.

Diese Ausführungsform hat den Vorteil, daß eine Anwendung auch bei solchen Tieren (und insbesondere Katzen) möglich ist, die eine Abneigung gegen den relativ ausgeprägten und intensiven Eigengeruch des Teebaumöls haben. Die Menge des Duftstoffes hängt von der Empfindlichkeit des Tieres ab. Im allgemeinen ist ein Zusatz (Zumischung) im Verhältnis von 1 bis 2 Vol% gegenüber 98 bis 99 Vol% Teebaumöl völlig ausreichend.

Die Wirkung dieser Stoffe beruht darauf, daß sie überraschenderweise bereits in dieser geringen Menge den Eigengeruch des Teebaumöls überdecken bzw. neutralisieren - jedenfalls soweit er für die Tiere unangenehm oder reizend ist - und die Wirksamkeit des Mittels in manchen Fällen sogar erhöhen.

Als besonders wirksam hat sich dabei der Zusatz von Cedernholzöl, Zitronenöl (DAB) oder Geraniumöl erwiesen, wobei ein Anteil von 1 bis 2 Vol% in den meisten Fällen ausreicht. Die Haltbarkeit dieses Arznei- bzw. Pflegemittels beträgt bei sachgemäßer und lichtgeschützter Lagerung etwa 60 Monate.

Durch Zusatz von Lavendelöl mit einem Anteil von etwa 5 Vol% gegenüber dem Teebaumöl erhält man ein Mittel, das besonders gut in die Haut eindringt und in den Blutkreislauf gelangt.

Als Spray, welches sowohl direkt auf das Tier, als auch in seine Umgebung (Käfig, Lagerstätten usw.) gesprüht werden kann, sowie zum Einreiben bei Entzündungen hat sich eine Emulsion des Mittels mit einem Anteil von 5 bis 10 Vol% in demineralisiertem Wasser mit einem Anteil von 90 bis 95 Vol% als besonders geeignet erwiesen, insbesondere wenn ein pflanzlicher Lebensmittelemulgator verwendet wird (Haltbarkeit des Mittels: etwa 48 Monate).

Ein pflanzlicher Lebensmittelemulgator ist deshalb vorteilhaft, weil er besonders gut verträglich ist und keine Nebenwirkungen hat, wie sie bei künstlichen (chemischen) Emulgatoren auftreten können.

Die Wirkungsweise beruht einerseits darauf, daß das Teebaumöl den Chitinpanzer insbesondere von Läusen zersetzt, während sich andererseits überraschend herausgestellt hat, daß der Emulgator die Atemwege der Parasiten verliebt und diese dadurch ersticken. Diese Wirkung tritt auch gegenüber Blattläusen an Pflanzen sowie Kopfläusen beim Menschen ein.

Ein weiterer Vorteil dieses Mittels besteht darin, daß es eine hervoragende pflegende und desinfizierende Wirkung hat und auch die Larven der oben genannten Parasiten abtötet und den Flohkot zersetzt.

Die der Erfindung zugrunde liegende Aufgabe wird ferner durch Arznei- bzw. Pflegemittel mit Teebaumöl für Tiere, insbesondere Haustiere, gemäß der Ansprüche 10 und 14 bis 16 gelöst, die neben den oben genannten Eigenschaften weitere vorteilhafte Anwendungen eröffnen:
Ein solches Mittel weist z.B. als Trägeröl Makadamia-Nußöl auf, welches ein reines Pflanzenöl ist, das aus der Makadamianuß (macadamia ternifolia) durch mechanische Erstpressung gewonnen wird.

Das Makadamia-Nußöl kann einen Anteil von etwa 90 Vol% aufweisen, ohne daß dadurch die Wirksamkeit des Teebaumöls wesentlich beeinträchtigt wird. Der Zusatz von Nußöl in dieser Menge hat darüberhinaus den Vorteil, daß es aufgrund seines mit über 20 Vol% sehr hohen Anteils an Palmitoleinsäure in Triglycerid-Form eine zusätzliche pflegende Wirkung entfaltet und ohne weitere Verarbeitung oder Zusatzstoffe dem Pflegemittel eine ölige Konsistenz (Pflegeöl) gibt, die leicht in die Tierhaut eindringt und eine sehr gute Spreitzung aufweist. Es ist bei sachgemäßer Lagerung in Lichtschutzflaschen mindestens 18 Monate haltbar.

Als Massageöl zur Behandlung von Rheumatismus, Arthritis, Verstauchungen und Liegeschwielen setzt sich das Mittel vorzugsweise aus einer Mischung von 80 Vol% Makadamia-Nußöl, 5 Vol% Teebaumöl, 10 Vol% Latschenkieferöl und 5 Vol% Bergamotteöl zusammen (Haltbarkeit ebenfalls etwa 18 Monate).

Insbesondere zur Ballenpflege hat sich eine Mischung aus 98 Vol% Makadamia-Nußöl mit 1 Vol% Teebaumöl und 1 Vol% Salbei als besonders geeignet erwiesen.

Diese Substanz kann z.B. in Lichtschutzflaschen mit Rollerkugeln abgefüllt werden und ist etwa 18 Monate haltbar.

Eine vorteilhafte Ausführungsform des Mittels, welches insbesondere auch zur Reinigung und Behandlung der Ohren z.B. bei bakteriellen Infekten geeignet ist, besteht aus einer Mischung von etwa 85 Vol% Makadamia-Nußöl und 10 Vol% Teebaumöl, dem weitere 3 Vol% Basilikumöl und 2 Vol% eines beliebigen anderen, als Duftstoff wirkenden ätherischen Öls zugesetzt sind. Es läßt sich mit einem Wattestäbchen gut anwenden. Die Haltbarkeit beträgt etwa 18 Monate.

Damit laßt sich auch dem sog. Ohrenzwang vorbeugen. Dies ist eine der häufigsten Ohrenkrankheiten, die besonders bei langhaarigen Rassen auftritt und durch Schmutzansammlungen im Ohr verursacht wird. Im ungünstigen Fall können sich dort sogar Ohrmilben entwickeln. Sofern dies bereits geschehen ist, können diese durch eine etwa zweiwöchige Behandlung mit so weit wie möglich (wiederum entsprechend der Empfindlichkeit des Tieres) reinem Teebaumöl (d.h. ohne Zusatzstoffe) wieder beseitigt werden. Allerdings sollte in diesem Fall unbedingt ein Tierarzt aufgesucht werden, der die weiter innen liegenden Teile des Gehörganges reinigt, da diese Bereiche leicht verletzt werden können.

Wird stattdessen als Darreichungsform eine Salbe benötigt, so hat sich der Einsatz von Wollwachs (DAB schadstoffarm) als Trägersubstanz als besonders vorteilhaft erwiesen. Wollwachs ist eine wachsartige, wasserfreie und gereinigte Substanz, die aus der Wolle des Schafes (ovis aries) gewonnen wird und höchstens 200 ppm Butylhydroxytoluol enthalten darf. Ein solches erfindungsgemäßes Arznei- bzw. Pflegemittel weist etwa 88 bis 90 Vol% Wollwachs, 8 bis 10 Vol% Teebaumöl und - bei empfindlicheren Tieren zur Geruchsneutralisierung - vorzugsweise 2 Vol% eines der oben genannten ätherischen Öle als Duftstoffe auf. Die Haltbarkeit beträgt bei lichtgeschützter Lagerung in randbefüllten Behältern mindestens 18 Monate.

Die Herstellung erfolgt in der Weise, daß das Wollwachs auf 50°C erwärmt und dann Teebaumöl beigemischt wird. Vor dem Erkalten werden die Substanzen nochmals gemischt (Haltbarkeit etwa 18 Monate). Wenn eine besonders weiche Konsistenz gewünscht wird, werden die Substanzen alternativ dazu zusammen solange vermischt, bis die entsprechende Konsistenz erreicht ist (Haltbarkeit etwa 12 Monate).

Alle Darreichungsformen der erfindungsgemäßen Arznei- bzw. Pflegemittel sollen in Lichtschutzflaschen oder -behältern aufbewahrt werden.

Ein weiteres erfindungsgemäßes Arznei- bzw. Pflegemittel mit Teebaumöl ist eine Emulsion mit einem Anteil von etwa 92 Vol% demineralisiertem Wasser mit einem pflanzlichen Lebensmittelemulgator, einem Anteil von 5 Vol% Teebaumöl und 3 Vol% Zitronenöl (DAB). Dieses Mittel hat den Vorteil, daß es insbesondere zur Reinigung und Pflege der Zähne und des Zahnfleisches sowie als Zahnbelagentferner geeignet ist. Es wird mit einem Pinsel aufgetragen wird. Der Pinsel kann z.B. am Schraubdeckel der Flasche befestigt sein. Das Zitronenöl hat in diesem Fall nicht nur die Funktion eines Duftstoffes, sondern unterstützt auch die Wirkung des Teebaumöls bei der Zahnbelagentfernung entscheidend. Bei sachgemäßer Lagerung beträgt die Haltbarkeit etwa 48 Monate.

Ein erfindungsgemäßes Arznei- bzw. Pflegemittel mit Teebaumöl für Tiere ist schließlich auch eine Emulsion mit einem Anteil von etwa 90 Vol% demineralisiertem Wasser mit einem pflanzlichen Lebensmittelemulgator und etwa 5 Vol% Teebaumöl, etwa 2 Vol% Lavendelöl und etwa 2 Vol% Geraniumöl. Dieses Mittel ist besonders vorteilhaft auch zur Reinigung und Pflege des äußeren Gehörgangs und der Ohrmuschel von Tieren geeignet.

Im folgenden sollen weitere Einzelheiten und vorteilhafte Anwendungen der oben beschriebenen Arznei- bzw. Pflegemittel erläutert werden:
Der Befall mit Flöhen und anderen blutsaugenden Parasiten, bei denen es sich nicht um Insekten im klassischen Sinn wie z.B. Fliegen und Mücken handelt, ist zum Teil weit verbreitet und unangenehm für Mensch und Tier. Es gibt insgesamt etwa 300 Floharten, von denen einige (z.B. pulex irritans) sowohl Hunde und Katzen, als auch Menschen befallen. Der Floh lebt blutsaugend auf seinem Wirt und kann nicht nur juckende Hautveränderungen verursachen, sondern auch Bandwürmer übertragen. Da nur ein geringer Teil der Flöhe direkt an seinem Wirt sitzt und sich insbesondere Eier und Larven in der Umgebung entwickeln, bevor sie wieder einen Wirt befallen, muß zur wirksamen Flohbekämpfung auch für eine flohfreie Umgebung gesorgt werden. Für Haustiere sind zahlreiche chemische Mittel wie Umgebungs- und Direktsprays sowie Flohhalsbänder auf Gas- oder Puderbasis bekannt, die jedoch aus naheliegenden Gründen dann abgelehnt werden, wenn kleine Kinder mit den Tieren spielen.

Mit den erfindungsgemäßen Mitteln ist eine wesentlich schonendere Vorbeugung gegen einen Befall mit Flöhen, Zecken, Milben, Haarlingen, Läusen und anderen blutsaugenden Parasiten sowie eine Beseitigung dieser Parasiten möglich.

Bei Hunden und Katzen hat es sich zur Vorbeugung als sehr wirksam erwiesen, wenn drei Wochen lang täglich links und rechts am Gehöreingang (weil fellfrei und gut durchblutet) einige Tropfen Teebaumöl einmassiert werden. Gegebenenfalls werden dem Teebaumöl etwa 1 bis 2 Vol% eines ätherischen Duftöls, vorzugsweise Zitronenöl zugesetzt. Das Mengenverhältnis hängt wiederum von der Empfindlichkeit des Tieres gegenüber dem Geruch des Teebaumöl ab und wird individuell bestimmt. Während Katzen meist relativ empfindlich auf den Geruch des Teebaumöl reagieren und somit einen größeren Anteil des Duftöls benötigen, kann bei Hunden im Idealfall reines Teebaumöl angewandt werden. Im Anschluß an diese drei Wochen braucht dieser Vorgang nur noch an ein bis zwei Tagen in der Woche wiederholt zu werden, um das Tier flohfrei zu halten.

Ist das Tier bereits mit Flöhen befallen, so wird es zunächst gründlich mit einem entsprechenden Shampoo mit nachfettender Wirkung gewaschen. Anschließend wird es in den Achselhöhlen, im Nacken und am Rutenansatz - mit dem gleichen Mittel wie oben unter Vorbeugung beschrieben - eingerieben. Zusätzlich müssen dann noch die Liegestätten und Aufenthaltsorte des Tieres mit dem Teebaumöl-Umgebungsspray eingesprüht werden. Die Einwirkzeit beträgt etwa 10 Minuten.

Diese Behandlung hat sich auch zur Linderung des Juckreizes infolge einer Futtermittelallergie bewährt. Die entsprechenden Stellen können sowohl mit dem Pflegeöl als auch mit der Teebaumöl-Salbe (bei bereits aufgekratzten Stellen) eingerieben werden.

Ebenfalls verbreitet und für Mensch und Tier gleichermaßen unangenehm und gefährlich sind Zecken (Holzbock), die sich in die Haut bohren, Blut saugen und zu Hautreizungen, Hauterkrankungen, Blutarmut, Kräfteverfall und sogar Gehirnhautentzündung führen können. Bei einem Zeckenbefall wird ein Tropfen möglichst reines Teebaumöl (entsprechend der Empfindlichkeit des befallenen Tieres) auf die Zecke gegeben. Nach etwa 2 Minuten läßt sich die Zecke nun mit einer Pinzette oder der Hand leicht entfernen. Um eine Entzündung der Bißstelle zu vermeiden, wird schließlich etwas Teebaumöl (möglichst rein) mit einem Wattestäbchen o.ä. aufgetupft.

Ein tägliches einmaliges Abwaschen mit einer Emulsion gemäß Anspruch 8 hat sich auch als vorbeugende Maßnahme gegen Entzündungen im Analbereich, die häufig auf einer Verstopfung der Analdrüsen beruhen, als wirksam erwiesen. Sofern die Entzündung bereits eingetreten ist, läßt sie sich mit dreimaligem täglichen Abwaschen gut lindern.

Ein Spray gemäß Anspruch 8 eignet sich hervorragend zur Desinfektion von Käfigen und Zwingern in Tierheimen, wobei zur Erhöhung der Wirkung 10 %iges reines Teebaumöl zusätzlich beigemischt werden kann. In normalen Wohnräumen ist dies jedoch nicht erforderlich. Die Einwirkungszeit sollte etwa eine Stunde betragen. Eventuelle Rückstände des Sprays brauchen nicht entfernt zu werden.

Mit einem Mittel gemäß Anspruch 10 können Penis- und Scheidenentzündungen durch großflächiges Einreiben morgens und mittags behandelt werden. Abends wird vorzugsweise die Pflegesalbe gemäß Anspruch 14 aufgetragen.

Mit dem Makadamia-Pflegeöl gemäß Anspruch 10 läßt sich durch Auftragen auf das gesamte Fell (nach gründlichem Waschen) auch eine Fellverfilzung mit Kamm oder Bürste leicht lösen.

Das Mittel gemaß Anspruch 11 ist auch bei Rheumatismus, Arthritis, Verstauchungen und Liegeschwielen wirksam, insbesondere wenn es erwärmt und an den schmerzenden Stellen einmassiert wird.

Zur Vermeidung und Linderung einer übermäßigen Schuppenbildung hat sich eine Beimischung von etwa 10 Tropfen reinen Teebaumöls zu einem Tiershampoo bewährt, welches man vor dem Ausspülen etwa 5 Minuten einwirken läßt.

Eine Pflegesalbe gemäß Anspruch 14 hat sich auch bei Hautentzündungen und Hautausschlägen als wirksam erwiesen, mit der die entsprechenden Stellen morgens und abends eingerieben werden.

Eine weitere überraschende Anwendung hat sich schließlich bei besonders nervösen oder unruhigen Tieren ergeben. In diesem Fall gibt man in eine mit Wasser gefüllte Duftlampe 4 bis 5 Tropfen Teebaumöl sowie die gleiche Menge eines anderen ätherischen Öles, welches für das Tier besonders angenehm duftet. Dies stellt man dadurch fest, daß man jeweils einen Tropfen verschiedener solcher Öle auf den Finger gibt und das Tier daran riechen läßt. Aus der Reaktion kann man dann erkennen, welcher Duft ihm besonders zusagt. Das mit der gesamten Mischung in einer Duftlampe freigesetzte Aroma hat dann eine sehr beruhigende Wirkung auf das Tier (Aroma-Therapie).

## Patentansprüche

1. Verwendung von Teebaumöl (Tea Tree) zur Herstellung eines Arznei- bzw. Pflegemittels zur Vorbeugung gegen und zur Behandlung bei einen/m Befall mit Flöhen, Zecken, Milben, Haarlingen, Läusen, und anderen blutsaugenden Parasiten bei Tieren, insbesondere Haustieren wie Hunden und Katzen,
dadurch gekennzeichnet, daß es Teebaumöl in reiner Form zur äußeren Anwendung aufweist.

2. Verwendung von Teebaumöl nach Anspruch 1,
dadurch gekennzeichnet, daß der Gehalt des Cineol 1,8 unter 4 Vol% und der Gehalt des Terpinen-4-ol über 34 Vol% liegt.

3. Verwendung von Teebaumöl nach Anspruch 1 oder 2,
dadurch gekennzeichnet, daß ein als Duftstoff wirkender, geringer Anteil eines anderen ätherischen Öles zugemischt wird.

4. Verwendung von Teebaumöl nach Anspruch 3,
dadurch gekennzeichnet, daß der Duftstoff Cedernholzöl oder Zitronenöl (DAB) ist.

5. Verwendung von Teebaumöl nach Anspruch 3,
dadurch gekennzeichnet, daß der Duftstoff Geraniumöl ist.

6. Verwendung von Teebaumöl nach einem der Ansprüche 3 bis 5,
dadurch gekennzeichnet, daß der Anteil des Duftstoffs etwa 1 bis 5 Vol% gegenüber dem Teebaumöl mit einem Anteil von etwa 95 bis 99 Vol% beträgt.

7. Verwendung von Teebaumöl nach einem der Ansprüche 1 bis 5,
dadurch gekennzeichnet, daß Lavendelöl mit einem Anteil von etwa 5 Vol% und ein Duftstoff mit einem Anteil von etwa 1 Vol%, jeweils gegenüber dem Teebaumöl zugemischt wird.

8. Verwendung von Teebaumöl zur Herstellung eines Arznei- bzw. Pflegemittels insbesondere zur Vorbeugung gegen und zur Behandlung bei einen/m Befall mit Flöhen, Zecken, Milben, Haarlingen, Läusen, und anderen Parasiten sowie Entzündungen bei Tieren, insbesondere Haustieren, zur Vorbeugung und Bekämpfung von Blattläusen und anderen Parasiten an Pflanzen, sowie von Kopfläusen beim Menschen,
dadurch gekennzeichnet, daß es eine Emulsion zum Versprühen oder Einreiben mit einem Anteil von etwa 90 bis 95 Vol% demineralisiertem Wasser mit einem pflanzlichen Lebensmittelemulgator sowie etwa 5 bis 10 Vol% Teebaumöl ist.

9. Arznei- bzw. Pflegemittel,
dadurch gekennzeichnet, daß es nach einem der Ansprüche 1 bis 8 hergestellt ist.

10. Arznei- bzw. Pflegemittel mit Teebaumöl für Tiere, insbesondere Haustiere,
dadurch gekennzeichnet, daß es ein Pflegeöl mit einem Anteil von etwa 90 Vol% Makadamia-Nußöl als Trägerstoff ist.

11. Arznei- bzw. Pflegemittel nach Anspruch 10,
dadurch gekennzeichnet, daß es etwa 5 Vol% Teebaumöl, etwa 80 Vol% Makadamia-Nußöl, etwa 10 Vol% Latschenkieferöl und etwa 5 Vol% Bergamotteöl aufweist.

12. Arznei- bzw. Pflegemittel nach Anspruch 10,
dadurch gekennzeichnet, daß es etwa 1 Vol% Teebaumöl, etwa 1 Vol% Salbei und etwa 98 Vol% Makadamia-Nußöl aufweist.

13. Arznei- bzw. Pflegemittel nach Anspruch 10,
dadurch gekennzeichnet, daß es etwa 10 Vol% Teebaumöl, etwa 85 Vol% Makadamia-Nußöl, etwa 3 Vol% Basilikumöl und etwa 2 Vol% eines anderen ätherischen Öles als Duftöl aufweist.

14. Arznei- bzw. Pflegemittel mit Teebaumöl für Tiere, insbesondere Haustiere,
dadurch gekennzeichnet, daß es eine Pflegesalbe mit einem Anteil von etwa 88 bis 90 Vol% Wollwachs (DAB schadstoffarm) als Trägerstoff ist.

15. Arznei- bzw. Pflegemittel mit Teebaumöl für Tiere, insbesondere Haustiere,
dadurch gekennzeichnet, daß es eine Emulsion mit einem Anteil von etwa 92 Vol% demineralisiertem Wasser mit einem pflanzlichen Lebensmittelemulgator, etwa 5 Vol% Teebaumöl und etwa 3 Vol% Zitronenöl ist.

16. Arznei- bzw. Pflegemittel mit Teebaumöl für Tiere, insbesondere Haustiere,
dadurch gekennzeichnet, daß es eine Emulsion mit einem Anteil von etwa 91 Vol% demineralisiertem Wasser mit einem pflanzlichen Lebensmittelemulgator ist und etwa 5 Vol% Teebaumöl, etwa 2 Vol% Lavendelöl und etwa 2 Vol% Geraniumöl aufweist.

17. Verwendung von Teebaumöl zur Herstellung eines Arznei- bzw. Pflegemittels nach einem der Ansprüche 10 bis 16.
